# EUROPEAN PATENT APPLICATION

(11) **EP 4 029 572 A1**
(43) Date of publication of application: **20.07.2022**
(21) Application number: 20862265.4
(22) Date of filing: 08.09.2020
(51) Int. Cl.: A61Q 1/04, A61Q 1/10, A61Q 1/12, A61Q 15/00, A61Q 17/04, A61Q 19/00, A61Q 19/10, A61K 8/02, A61K 8/19

(54) **POWDER SURFACE TREATMENT METHOD, SURFACE TREATING AGENT COMPOSITION USED THEREFOR, AND SURFACE-TREATED POWDER**

(30) Priority: 13.09.2019 JP 2019167238
(71) Applicant: AJINOMOTO CO., INC., Chuo-ku Tokyo 104-8315 (JP)
(72) Inventor: WAKUI, Wataru, Kawasaki-shi, Kanagawa 210-8681 (JP)
(74) Representative: Mewburn Ellis LLP
(86) International application number: PCT/JP2020/033943
(87) International publication number: WO 2021/049483

(57) **Abstract**

The present invention provides a powder surface treatment method comprising (i) a step for mixing 0.001-20 parts by mass of a surface treating agent composition with 100 parts by mass of a power, and (ii) a step for adding an acidic liquid, followed by mixing, wherein the surface treating agent composition contains (a)-(d) below: (a) one or more selected from among an N-acyl amino acid having a carbon chain length of C8-C22 and a salt thereof; (b) one or more selected from among monohydric and polyhydric alcohols; (c) water; and (d) one or more selected from among (dl) an amino acid having an isoelectric point of 7.5-11, and (d2) a compound which generates an ion selected from among an aluminum ion, a magnesium ion, a calcium ion, a zinc ion, a zirconia ion, and a titanium ion, in the surface treating agent composition.

## Description

### Technical Field

The present invention relates to a powder surface treatment method, a surface treating agent composition used therefor, and a surface-treated powder. Further, the present invention relates to a cosmetic containing the surface-treated powder.

### Background Art

Powders used in cosmetics are subjected to various surface treatments to improve their feel (such as smoothness, elongation, moistness, dehydration, and adhesion when applied to the skin), cosmetic effects (such as coverage, soft focus, light absorption and dispersion, and coloration), long-lasting makeup (such as prevention of dullness and discoloration caused by wetting of the powder with sweat and sebum, adhesiveness, and resistance to running and patchiness), formulation stability (dispersibility and emulsion stability), and formability. Since the required effects vary depending on the application, many surface treating agents have been proposed so far.

As an example, the surface treatment of powders with N-acyl amino acids has been attempted for a long time, and surface treatment methods using an aluminum salt, a magnesium salt, a calcium salt, a zinc salt, a zirconia salt, or a titanium salt of N-acyl amino acid have been disclosed (Japanese Patent Application Publication No. Sho 58-72512 (Patent Literature 1)). In order to maintain the state of adhesion to the surface even after an N-acyl amino acid is blended into a cosmetic or the like containing water and oil, it is not enough to simply mix the N-acyl amino acid and the powder. In Patent Literature 1, a divalent or higher-valent metal ion with positive charge is used to bind the hydroxyl group, which is a negative charge on the powder surface, to the carboxyl group, which is a negative charge on the N-acyl amino acid, and is adsorbed in an oriented manner in water or a solvent aqueous solution. This method has little industrial advantage because it requires the steps of filtration, washing, drying, and grinding. In addition, because of the need for drying and grinding, the powder may easily agglomerate and the oil dispersibility may be insufficient. Further, the burden on the environment is also great.

On the other hand, there is also known a modification method in which plasma is applied to the powder to be treated before surface treatment and the surface is directly reacted with an acyl amino acid (Japanese Patent Application Publication No. 2014-19783). This method requires special equipment such as electrodes to generate plasma.

### Summary of Invention

An object of the present invention is to provide a simple method for surface treatment of a powder, which can be carried out with a small number of man-hours and does not require a special device. Another object of the present invention is to provide a powder having improved performance as compared with conventional products by the treatment method, particularly a powder having excellent oil dispersibility.

The present inventors have made earnest studies, and as a result have found for the first time that the above objects can be achieved by using a specific amount of alcohol, water, and an amino acid having an isoelectric point of 7.5 to 11 or a specific ion. Thus, the present invention has been completed based on this fact. Specifically, the present invention is as follows.
[1] A powder surface treatment method comprising the steps of:
   (i) mixing 0.001 to 20 parts by mass of a surface treating agent composition based on 100 parts by mass of a powder; and
   (ii) adding an acidic liquid, followed by mixing, wherein
      the surface treating agent composition contains the following components (a) to (d)
      (a) at least one selected from N-acyl amino acids having a carbon chain length of C8 to C22 and salts thereof,
      (b) at least one selected from monohydric and polyhydric alcohols,
      (c) water, and
      (d) at least one selected from the following (d1) and (d2)
         (d1) amino acids having an isoelectric point of 7.5 to 11 and
         (d2) compounds that produce ions selected from aluminum ions, magnesium ions, calcium ions, zinc ions, zirconia ions, and titanium ions in the surface treating agent composition.
[2] The method according to [1], wherein in the step (i), the surface treating agent composition is mixed by dropping or mixed by spraying.
[3] The method according to [1] or [2], wherein in the step (i), the surface treating agent composition is mixed in an amount of 0.1 to 20 parts by mass based on 100 parts by mass of the powder.
[4] The method according to any one of [1] to [3], wherein in the steps (i) and (ii), the mixing is performed using a mixer selected from the group consisting of high-speed stirring type mixers, container rotary type mixers, container rotary type mixers with a stirrer, mechanical stirring type mixers, airflow stirring type mixers, and compression/shear/impact type mixers.
[5] The method according to any one of [1] to [4], wherein the acidic liquid is a citric acid aqueous solution or sulfuric acid.
[6] The method according to any one of [1] to [5], wherein in the step (ii), the acidic liquid is mixed in an amount of 0.01 to 15 parts by mass based on 100 parts by mass of the powder.
[7] The method according to any one of [1] to [6], wherein the powder is at least one selected from the group consisting of resin powders, silicon-containing powders, metal oxides, carbon-containing powders, fluorine-containing powders, metal salts, boron-containing powders, composite powder crystals, and non-crystalline powders.
[8] The method according to any one of [1] to [7], which is free of a drying step using power and an apparatus.
[9] A surface treating agent composition comprising the following components (a) to (d):
   (a) at least one selected from N-acyl amino acids having a carbon chain length of C8 to C22 and salts thereof;
   (b) at least one selected from monohydric and polyhydric alcohols;
   (c) water; and
   (d) at least one selected from the following (d1) and (d2)
      (d1) amino acids having an isoelectric point of 7.5 to 11 and
      (d2) compounds that produce ions selected from aluminum ions, magnesium ions, calcium ions, zinc ions, zirconia ions, and titanium ions in the surface treating agent composition.
[10] The surface treating agent composition according to [9], wherein a transmittance at a wavelength of 660 nm is 80% or more.
[11] The surface treating agent composition according to [9] or [10], wherein a concentration of (a) in the surface treating agent composition is 4 to 25% by mass.
[12] The surface treating agent composition according to any one of [9] to [11], wherein a concentration of (b) in the surface treating agent composition is 3 to 80% by mass.
[13] The surface treating agent composition according to any one of [9] to [12], wherein a concentration of (c) in the surface treating agent composition is 3 to 80% by mass.
[14] The surface treating agent composition according to any one of [9] to [13], wherein a concentration of (d) in the surface treating agent composition is 0.5 to 15% by mass.
[15] The surface treating agent composition according to any one of [9] to [14], wherein a solid content concentration in the surface treating agent composition is 10 to 23% by mass.
[16] The surface treating agent composition according to any one of [9] to [15], wherein (a) in the surface treating agent composition includes at least one selected from myristoyl glutamic acid, sodium myristoyl glutamate, palmitoylglutamic acid, sodium palmitoylglutamate, stearoyl glutamic acid, sodium stearoyl glutamate, disodium stearoyl glutamate, and sodium oleoyl glutamate.
[17] The surface treating agent composition according to any one of [9] to [16], wherein (b) in the surface treating agent composition includes at least one selected from ethanol, 2-propanol, glycerin, pentanediol, dipropylene glycol, 1,3-butylene glycol, and pentylene glycol.
[18] The surface treating agent composition according to any one of [9] to [17], wherein (d) in the surface treating agent composition includes at least one amino acid selected from histidine, lysine, and arginine.
[19] The surface treating agent composition according to any one of [9] to [18], further comprising: at least one selected from sodium hydroxide, potassium hydroxide, sodium carbonate, potassium carbonate, and sodium hydrogen carbonate.
[20] The surface treating agent composition according to any one of [9] to [19], further comprising: at least one selected from fatty acids having a carbon chain length of C8 to C22 and salts thereof.
[21] A powder treated by the method according to any one of [1] to [8].
[22] A cosmetic powder comprising, on a surface thereof:
   (a) at least one selected from N-acyl amino acids having a carbon chain length of C8 to C22 and salts thereof; and
   (d1) at least one selected from amino acids having an isoelectric point of 7.5 to 11.
[23] The cosmetic powder according to [22], further comprising, on the surface thereof: (b) at least one selected from monohydric and polyhydric alcohols.
[24] A cosmetic composition comprising: the powder according to any one of [21] to [23].

The powder obtained by the method of the present invention is particularly excellent in oil dispersibility, has a moist feel, is easily dispersed in an oily formulation, and has an effect of not running down by sebum or sweat.

### Description of Embodiments

A powder surface treatment method of the present invention includes the following steps:
(i) mixing 0.001 to 20 parts by mass of a surface treating agent composition based on 100 parts by mass of a powder; and
(ii) adding an acidic liquid to the powder mixed in the step (i), followed by mixing.

Here, the surface treating agent composition contains the following components (a) to (d)
(a) at least one selected from N-acyl amino acids having a carbon chain length of C8 to C22 and salts thereof,
(b) at least one selected from monohydric and polyhydric alcohols,
(c) water, and
(d) at least one selected from the following (d1) and (d2)
   (d1) amino acids having an isoelectric point of 7.5 to 11 and
   (d2) compounds that produce ions selected from the group consisting of aluminum ions, magnesium ions, calcium ions, zinc ions, zirconia ions, and titanium ions in the surface treating agent composition.

As for the component (a) contained in the surface treating agent composition of the present invention, the amino acid component constituting the N-acyl amino acid having a carbon chain length of C8 to C22 may be any of acidic, neutral, and basic amino acids, and may be any of α, β, and ε-amino acids. Examples thereof include glycine, β-alanine, α-alanine, proline, valine, leucine, phenylalanine, 3,4-dioxyphenylalanine, serine, threonine, methionine, lysine, ornithine, arginine, histidine, ε-aminocaproic acid, glutamic acid, aspartic acid, and the like. Glycine, β-alanine, α-alanine, proline, threonine, lysine, arginine, glutamic acid, and aspartic acid are more preferable, glycine, β-alanine, α-alanine, threonine, glutamic acid, and aspartic acid are more preferable, and glutamic acid and aspartic acid are further preferable.

In the case of using an amino acid component having multiple amino groups (including imino groups), it is sufficient that at least one of the amino groups is acylated, and for example, all of the amino groups may be acylated with multiple kinds of acyl components, or may be in the form of a mono-N-acyl derivative.

The acyl component constituting the N-acyl group of the N-acyl amino acid having a carbon chain length of C8 to C22 is an acyl group that is or can be derived from linear or branched chain, saturated or unsaturated fatty acids having 8 to 22 carbon atoms, such as single fatty acid acyl groups such as octanoyl groups, caproyl groups, nonanoyl groups, caprinoyl groups, decanoyl groups, undecanoyl groups, lauroyl groups, myristoyl groups, palmitoyl groups, stearoyl groups, behenoyl groups, palmitoleoyl groups, oleoyl groups, and linoleoyl groups, and natural mixed fatty acid acyl groups such as coconut oil fatty acid acyl and hardened beef tallow fatty acid acyl, as well as aromatic carboxylic acid acyl groups such as benzoate acyl group. Such an acyl group can be derived from a fatty acid, but it can also be similarly derived from a raw material substance other than a fatty acid (such as fatty acid ester, fatty acid salt, acid halide, or acid anhydride). N-acyl amino acids having a carbon chain length of C8 to C20 are preferable, and N-acyl amino acids having a carbon chain length of C14 to C18 are more preferable. Myristoyl groups, palmitoyl groups, stearoyl groups, behenoyl groups, palmitoyl groups, oleoyl groups, and linoleoyl groups are preferable, myristoyl groups, palmitoyl groups, and stearoyl group are more preferable, and palmitoyl groups and stearoyl groups are even further preferable.

The salt of N-acyl amino acid having a carbon chain length of C8 to C22 includes pharmacologically acceptable salts and the like, which include alkali metal salts such as lithium salts, sodium salts, and potassium salts; alkaline earth metal salts such as calcium salts and magnesium salts; ammonium salts; and basic organic salts, triethanolamine salts, and the like. Of these, from the viewpoint of solubility, sodium salts, potassium salts, and ammonium salts are preferable, sodium salts and potassium salts are more preferable, and sodium salts are further preferable. Further, in the case of a polybasic acid such as a dibasic acid, both a monosalt (such as monosodium glutamate) and a disalt (such as disodium glutamate) can be used.

Specific examples of the N-acyl amino acid having a carbon chain length of C8 to C22 or salts thereof include myristoyl glutamic acid, sodium myristoyl glutamate, palmitoylglutamic acid, sodium palmitoylglutamate, stearoyl glutamic acid, sodium stearoyl glutamate, disodium stearoyl glutamate, sodium oleoyl glutamate, and the like, preferably myristoyl glutamic acid, sodium myristoyl glutamate, palmitoylglutamic acid, sodium palmitoylglutamate, stearoyl glutamic acid, sodium stearoyl glutamate, disodium stearoyl glutamate, and sodium oleoyl glutamate. By using these N-acyl amino acids, the oil dispersibility of the treatment powder can be improved.

The concentration of the component (a) in the surface treating agent composition is preferably 4 to 25% by mass, and more preferably 7 to 20% by mass. By setting the concentration of the component (a) in the surface treating agent composition to such a concentration, the oil dispersibility of the treatment powder can be improved even when the surface treating agent composition is used in a small amount.

Examples of the monohydric or polyhydric alcohol which is the component (b) contained in the surface treating agent composition of the present invention include ethanol, 2-propanol, glycerin, pentanediol, dipropylene glycol, 1,3-butylene glycol, pentylene glycol, ethylene glycol, propylene glycol, diethylene glycol, triethylene glycol, trimethylolpropane, triethanolamine, pentaerythritol, diglycerin, xylose, sorbitan, xylitol, triglycerin, glucose, fructose, sorbitol, malbitol, tetraglycerin, polyglycerin, sucrose, trehalose, lactose, maltotriose, and the like, preferably ethanol, 2-propanol, glycerin, pentanediol, dipropylene glycol, 1,3-butylene glycol, and pentylene glycol, more preferably glycerin, pentanediol, dipropylene glycol, 1,3-butylene glycol, and pentylene glycol, further preferably glycerin, pentanediol, dipropylene glycol, and 1,3-butylene glycol, and even further preferably glycerin. By using a monohydric or polyhydric alcohol, the solubility of the component (a) in the surface treating agent composition can be further increased, and the surface treating agent composition can be used in a small amount.

The concentration of the component (b) in the surface treating agent composition is preferably 3 to 80% by mass, more preferably 5 to 75% by mass, or can be 10 to 70% by mass, and further preferably 16 to 67% by mass. By setting the concentration of the component (b) in the surface treating agent composition to such a concentration, the oil dispersibility of the treatment powder can be improved.

Further, the concentration of the component (c) in the surface treating agent composition is preferably 3 to 80% by mass, more preferably 5 to 75% by mass, or can be 10 to 70% by mass, and further preferably 12 to 67% by mass. By setting the concentration of the component (c) in the surface treating agent composition to such a concentration, the oil dispersibility of the treatment powder can be improved.

The amino acid having an isoelectric point of 7.5 to 11, which is the component (d1) contained in the surface treating agent composition of the present invention, includes histidine (isoelectric point: 7.59), lysine (isoelectric point: 9.75), and arginine (isoelectric point: 10.76).

The compound which is the component (d2) contained in the surface treating agent composition of the present invention is not particularly limited as long as it can generate ions selected from the group consisting of aluminum ions, magnesium ions, calcium ions, zinc ions, zirconia ions, and titanium ions. Examples of such compound include aluminum hydroxide, aluminum sulfate, aluminum chloride, aluminum nitrate, sodium aluminate, potassium aluminum sulfate, magnesium hydroxide, magnesium chloride, magnesium sulfate, magnesium nitrate, potassium magnesium sulfate, calcium hydroxide, calcium chloride, calcium nitrate, calcium acetate, zinc chloride, zinc nitrate, zinc sulfate, zinc acetate, zirconium sulfate, zirconium chloride, titanium oxysulfate, titanium tetrachloride, and the like.

The component (d) contained in the surface treating agent composition of the present invention preferably includes at least one amino acid selected from histidine, lysine, and arginine. By using these amino acids, the oil dispersibility of the treatment powder can be improved.

The concentration of the component (d) in the surface treating agent composition is preferably 0.5 to 15% by mass, more preferably 0.7 to 10% by mass, or can be 1.0 to 8% by mass, and further preferably 1.5 to 7% by mass. By setting the concentration of the component (d) in the surface treating agent composition to such a concentration, the oil dispersibility of the treatment powder can be improved.

The surface treating agent composition of the present invention may further contain at least one selected from sodium hydroxide, potassium hydroxide, sodium carbonate, potassium carbonate, and sodium hydrogen carbonate. By containing these, the hydroxyl groups on the surface of the powder to be treated are dissociated and the treatment can be performed efficiently. Its concentration in the surface treating agent composition is preferably 0.1 to 3% by mass, and more preferably 1 to 2.5% by mass.

Further, the surface treating agent composition of the present invention may further contain at least one selected from fatty acids having a carbon chain length of C8 to C22 and salts thereof. By containing at least one selected from fatty acids having a carbon chain length of C8 to C22 and salts thereof, the oil dispersibility of the treatment powder can be improved. Examples of the fatty acids having a carbon chain length of C8 to C22 include caprylic acid, capric acid, lauric acid, myristic acid, palmitic acid, palmitoleic acid, stearic acid, arachidic acid, behenic acid, oleic acid, linoleic acid, linolenic acid, eicosenoic acid, erucic acid, hardened beef tallow fatty acid, palm oil fatty acid, palm oil fatty acid, and the like. Fatty acids having a carbon chain length of C8 to C20 are preferable, and fatty acids having a carbon chain length of C14 to C18 are more preferable. In addition, examples of salts of the fatty acids having a carbon chain length of C8 to C22 include a pharmacologically acceptable salts and the like, which include alkali metal salts such as lithium salts, sodium salts, and potassium salts; alkaline earth metal salts such as calcium salts and magnesium salts; ammonium salts; and basic organic salts, triethanolamine salts, and the like. The concentration of the fatty acid having a carbon chain length of C8 to C22 and a salt thereof in the surface treating agent composition is preferably 0.1 to 15% by mass, and more preferably 0.5 to 2.5% by mass.

The surface treating agent composition of the present invention preferably has a transmittance of 60% or more, and more preferably 80% or more, at a wavelength of 660 nm. By setting the transmittance of the surface treating agent composition in such a range, the oil dispersibility of the treatment powder can be improved.

The solid content concentration in the surface treating agent composition is preferably 10 to 23% by mass, more preferably 12 to 22% by mass, and further preferably 14 to 20% by mass. By setting the solid content concentration in the surface treating agent composition in such a range, the surface treating agent can be treated on the treatment powder at a high density, and the oil dispersibility of the treatment powder can be improved.

In the step (i), the surface treating agent composition can be in an amount of 0.001 to 20 parts by mass, preferably in an amount of 0.01 to 20 parts by mass or 0.1 to 18 parts by mass, more preferably 1 to 15% by mass or 3 to 15% by mass, based on 100 parts by mass of the powder, and is further preferably mixed in an amount of 7 to 16 parts by mass. By using the surface treating agent composition in such an amount, the steps of washing, filtering, drying, and the like of the treatment powder can be omitted.

The powder used in the surface treatment method of the present invention is not particularly limited as long as it is used for industrial purposes or cosmetics (pigments, dyes, resins, pearls), and examples thereof include resin powders such as nylon powder, nylon beads, silicone beads, and polyethylene beads;
metal oxides such as iron oxide (yellow pigment), iron oxide (red pigment), iron oxide (black pigment), tin oxide, chromium oxide, cobalt oxide, zinc oxide, pigment-grade zinc oxide, titanium oxide, pigment-grade titanium oxide, zirconium oxide, aluminum oxide, cerium oxide, fine particle titanium oxide, ultra-fine particle titanium oxide, fine particle zinc oxide, and fine particle iron oxide;
silicon-containing powders such as silicates ((Al/Ca/Na) silicate and (Na/Mg) silicate), sericite, mica, talc, kaolin, bentonite, aluminum silicate, magnesium silicate, cubic Na aluminosilicate, silicon carbide, and silicon oxides such as hydrous silica and silicic anhydride (foliar silica, non-porous silica, porous silica, porous silica, and semi-porous silica);
metal fatty acid soaps such as Mg stearate, Mg myristate, and Zn stearate, carbon-containing powders such as cellulose, cellulose particles, starch, corn starch, rice starch, potato starch, wheat flour, wood powder, carbon black, graphite, ultramarine, Prussian blue, and carmine;
metal salts such as barium sulfate, plate barium sulfate, butterfly barium sulfate, calcium carbonate, and magnesium carbonate;
fluorine-containing powders such as synthetic phlogopite (synthetic mica) and synthetic phlogopite iron;
boron-containing powders such as boron nitride;
composite powders such as pearl powder, colored pearl pigments, and mica titanium; and wax, dyes, rake, and the like.

Further, the powder may be subjected to surface treatment such as silicone treatment, fluorine compound treatment, silane coupling agent treatment, silane treatment, organic titanate treatment, fatty acid treatment, metal soap treatment, oil preparation treatment, amino acid treatment, and the like.

Note that resin powders, silicon-containing powders, metal oxides, carbon-containing powders, fluorine-containing powders, metal salts, boron-containing powders, composite powder crystals, or non-crystalline powders are preferable in terms of improving water repellency and oil repellency after treatment.

The powder can preferably be, for example, talc, mica, sericite, titanium oxide, red iron oxide, yellow iron oxide, and black iron oxide.

In the step (ii), the acidic liquid is mixed in an amount of preferably 0.01 to 15 parts by mass, and in an amount of more preferably 0.1 to 10 parts by mass, based on 100 parts by mass of the powder. By using an acidic liquid in such an amount, the oil dispersibility of the treatment powder can be improved.

The acidic liquid used in the surface treatment method of the present invention includes citric acid aqueous solution, tartaric acid aqueous solution, lactic acid, malic acid aqueous solution, succinic acid aqueous solution, ascorbic acid aqueous solution, acetic acid, sulfuric acid, hydrochloric acid, phosphoric acid, boric acid, and the like, preferably citric acid aqueous solution and sulfuric acid. The use of these acidic liquids is preferable in that the surface treating agent is hard to separate from the surface of the powder, and when the treatment powder gets wet with water, the pH becomes close to that of human skin, resulting in a treatment powder that is less irritating to the skin.

The mixing of steps (i) and (ii) may be performed using a mixer selected from high-speed stirring type mixers such as a Henschel mixer, an FM mixer, a high-shear mixer, a vertical mixer, and a planetary mixer, container rotary type mixers or container rotary type mixers with a stirrer such as a W-type mixer, a CV-type mixer, a V-type mixer, a locking mixer, a container mixer, a bole mixer, and a container mixer with chopper, mechanical stirring type mixers such as a paddle mixer, ribbon stirring type, double-screw paddle type, biaxial planetary stirring type, a Nauta mixer, and a conical screw type, airflow stirring type mixers, and compression/shear/impact type mixers such as a Julia mixer and Nobilta.

Further, from the viewpoint of upscaling and the like, the surface treating agent composition and/or the acidic liquid may be mixed by dropping or mixed by spraying.

From the viewpoint of handling the treatment powder, the surface treatment method of the present invention is preferably free of a drying step using power and an apparatus.

The powder treated by the surface treatment method of the present invention is particularly excellent in oil dispersibility, has a moist feel, is easily dispersed in an oily formulation, and has an effect of not running down by sebum or sweat. Further, by using the powder treated by the surface treatment method of the present invention in the cosmetic composition, the color development of the pigment is improved (the color of the pigment becomes vivid).

Further, the cosmetic powder of the present invention has, on the surface thereof, (a) at least one selected from N-acyl amino acids having a carbon chain length of C8 to C22 and salts thereof and (d1) at least one selected from amino acids having an isoelectric point of 7.5 to 11. Here, "has, on the surface thereof" includes a mode in which the component (a) is simply adsorbed, as well as a mode in which the component (a) is bound via the component (d1). For example, it includes a mode in which the dissociated and negatively charged hydroxyl groups on the surface of titanium oxide as the powder to be treated ionically bond to the dissociated and negatively charged carboxyl groups of disodium N-stearoyl glutamate as the component (a) via the dissociated and positively charged amino groups of L-arginine as the component (d1). Further, the cosmetic powder of the present invention may further have, on the surface thereof, (b) at least one selected from monohydric and polyhydric alcohols. The monohydric and polyhydric alcohols are preferably ethanol, 2-propanol, glycerin, pentanediol, dipropylene glycol, 1,3-butylene glycol, and pentylene glycol, more preferably glycerin, pentanediol, dipropylene glycol, 1,3-butylene glycol, and pentylene glycol, further preferably glycerin, pentanediol, dipropylene glycol, and 1,3-butylene glycol, and even further preferably glycerin.

The cosmetic composition containing the powder treated by the surface treatment method of the present invention can be, for example, any form of cosmetic that can be applied to a desired site (for example, skin, hair, scalp, lips, eyes, eyelashes, eyelids, and nails) according to a conventional method. Examples of cosmetics for the skin, lips, eyelashes, and nails include suntan lotions such as sunscreens, body powders, and sprays, makeup cosmetics such as foundations, primers, body colors, bronzers, face powders, nail polishes, cheek colors, makeup bases, and concealers, lip cosmetics such as lip colors, lip liners, and lipsticks, eye makeup cosmetics such as eyeliners, eyeshadows, eyebrows, and mascaras, leave-on cosmetics such as emulsions, lotions, creams, gels, and beauty essences, and face masks. Examples of cosmetics for hair include hair styling products, hair emulsions, hair treatments, hair conditioners, and hair lotions. Examples of cosmetics for the scalp include hair restorers. Examples of preferable cosmetics include make-up cosmetics, eye makeup cosmetics, lip cosmetics, and leave-on cosmetics. Examples of preferable external agents include ointments, creams, mousses, and gels.

Next, the present invention is described with reference to Examples and Comparative Examples, but the present invention is not limited thereto.

### Examples

### <Example 1>

In a mixed solution of 1.2 g of glycerin and 1.2 g of water, 0.6 g of disodium N-stearoyl glutamate and 0.1 g of L-arginine hydrochloride were dissolved to obtain a surface treating agent composition. This surface treating agent composition was added to 20 g of titanium oxide (ISHIHARA SANGYO KAISHA, LTD., CR-50), and mixing for 20 seconds was repeated 3 times with Mill & Mixer (Tescom, TNIL161). Then, 0.56 g of a 50% (w/v) citric acid aqueous solution was added, and mixing for another 20 seconds was repeated 3 times to obtain titanium oxide surface-treated with disodium N-stearoyl glutamate without washing, filtering, or drying.

### <Comparative Example 1>

The method (wet method) described in Japanese Patent Application Publication No. Sho 58-72512 (Patent Literature 1):
In a 200 mL beaker, 0.6 g of disodium N-stearoyl glutamate was dissolved in 43.9 g of water at 60°C, and 20 g of titanium oxide (ISHIHARA SANGYO KAISHA, LTD., CR-50) was dispersed. To this solution, 0.1 g of sodium aluminate was added and dissolved. After continuing to stir for 30 minutes, 64% sulfuric acid was added until the pH of the solution reached 6. The obtained slurry was centrifuged at 5000 rpm for 5 minutes with a centrifuge (Hitachi Koki Co., Ltd., CR22G III), and then the supernatant was removed, and the same amount of water as the amount of the removed liquid was added and redispersed by centrifugation, which was repeated 4 times. Then, the resultant with the supernatant removed was dried in a constant temperature bath at 70°C for 48 hours, and ground for 20 seconds with Mill & Mixer (Tescom, TML161) 3 times to obtain titanium oxide surface-treated with disodium N-stearoyl glutamate.

### <Comparative Example 2>

### Simple mixing (dry method)

Titanium oxide (ISHIHARA SANGYO KAISHA, LTD., CR-50) at 20 g, disodium N-stearoyl glutamate (powder) at 0.6 g, and L-arginine hydrochloride at 0.1 g were mixed 3 times for 20 seconds with Mill & Mixer (Tescom, TML161) to obtain titanium oxide surface-treated with disodium N-stearoyl glutamate.

### <Evaluation>

Evaluation of the oil dispersibility of powder:
JIS K 5101-13-1: 2004 Test Methods for Pigments - Part 13: Oil Absorption - Section 1: Refined Linseed Oil Method was referred to. On a glass plate, 1 g of the powder to be evaluated was placed, silicone oil (Shin-Etsu Chemical Co., Ltd., SH245) was added, and the mixture was kneaded with a paint knife to form one unit. The amount of oil at this time was defined as the oil absorption point. The silicone oil was further added and kneaded, and the plate was tilted to allow the oil-absorbed powder to flow. The amount of oil at this time was defined as the pour point. The difference between the pour point and the oil absorption point was used as an index of dispersibility.

Based on the index of the powder before the surface treatment, the scores were determined as follows: 5 points if the index after the surface treatment was less than 30%, 3 points in the case of 30% or more and less than 50%, 2 points in the case of 50% or more and less than 70%, 1 point in the case of 71% or more, and 0 points in the case of 100 points or more.

Instead of silicone oil, the scores were also determined for a hydrocarbon (Moresco, liquid paraffin P-55), a triglyceride (KOKYU ALCOHOL KOGYO CO., LTD., TCG-M), and an ester oil (KOKYU ALCOHOL KOGYO CO., LTD., CEH).

The total score of the four oils was evaluated as A for 16 to 20 points, B for 12 to 15 points, C for 8 to 11 points, D for 4 to 7 points, and E for 3 points or less.

When the oil dispersibility of Example 1 and Comparative Examples 1 and 2 was evaluated, it was A for Example 1, D for Comparative Example 1, and E for Comparative Example 2. In order to fix the acyl amino acid on the surface of the powder, oriented adsorption is necessary, and fixture on the surface is impossible by simple mixing (Comparative Example 2). On the other hand, although the wet method (Comparative Example 1) as described in Japanese Patent Application Publication No. Sho 58-72512 (Patent Literature 1) is effective for oriented adsorption, it is not cost-effective due to steps such as washing and drying, and the oil dispersibility is inferior to the present method (Example 1) due to the low fixation rate on the surface.

The type of alcohol (b) in the surface treating agent composition was examined. To confirm the solubility of acyl amino acids in the surface treating agent composition, a 96-well plate in Grating Microplate Reader (CORONA ELECTRIC Co., Ltd., SH-1000) was used to measure the absorbance of the surface treating agent composition at a wavelength of 660 nm, and the converted transmittance was measured. Further, the oil dispersibility of the powder treated with the surface treatment composition was evaluated as described above.

### <Example 2>

In 1.2 g of ethanol and 1.2 g of water, 0.4 g of disodium N-stearoyl glutamate and 0.1 g of L-arginine hydrochloride were dissolved to obtain a surface treating agent composition. This surface treating agent composition was added to 20 g of titanium oxide (ISHIHARA SANGYO KAISHA, LTD., CR-50), and mixing for 20 seconds was repeated 3 times with Mill & Mixer (Tescom, TML161). Then, 0.56 g of a 50% (w/v) citric acid aqueous solution was added, and mixing for another 20 seconds was repeated 3 times to obtain titanium oxide surface-treated with disodium N-stearoyl glutamate without washing, filtering, or drying. Table 1 presents the results.

### <Examples 3 to 7>

Titanium oxide surface-treated with disodium N-stearoyl glutamate was obtained in the same manner as in Example 2 except that 1.2 g of ethanol in Example 2 was changed to the alcohols presented in Table 1. Table 1 presents the results.

**[Table 1]**

| | | Example/Comparative Example | Exampl e2 | Exampl e3 | Exampl e4 | Exampl e5 | Exampl e6 | Exampl e7 |
|---|---|---|---|---|---|---|---|---|
| Powder | | Titanium Oxide | 20 | 20 | 20 | 20 | 20 | 20 |
| Surface Treating Agent Composition | (a) | 2Na Stearoyl Glutamic Acid | 0.4 | 0.4 | 0.4 | 0.4 | 0.4 | 0.4 |
| | (b) | Ethanol | 1.2 | | | | | |
| | (b) | 2-Propanol | | 1.2 | | | | |
| | (b) | 1,3-Butylene Glycol | | | 1.2 | | | |
| | (b) | Dipropylene Glycol | | | | 1.2 | | |
| | (b) | Pentylene Glycol | | | | | 1.2 | |
| | (b) | Glycerin | | | | | | 1.2 |
| | (c) | Water | 1.2 | 1.2 | 1.2 | 1.2 | 1.2 | 1.2 |
| | (d) | L-Arg (Isoelectric Point 10.76) | 0.1 | 0.1 | 0.1 | 0.1 | 0.1 | 0.1 |
| Acidic Liquid | | 50% Citric Acid Liquid | 0.56 | 0.56 | 0.56 | 0.56 | 0.56 | 0.56 |
| Evaluation | | Evaluation of Oil Dispersibility | B | A | A | A | A | A |
| | | Transmittance of Surface Treating Agent Composition (Wavelength of 660 nm) | 92.5 | 92.9 | 88.5 | 91.8 | 92 | 90.6 |

### Examination of the type of amino acid (d1) in the surface treating agent composition

### <Example 8>

Titanium oxide surface-treated with disodium N-stearoyl glutamate was obtained in the same manner as in Example 7 except that 0.1 g of L-arginine hydrochloride in Example 7 was changed to 0.1 g of L-histidine. Table 2 presents the results.

### <Example 9>

Titanium oxide surface-treated with disodium N-stearoyl glutamate was obtained in the same manner as in Example 7 except that 0.1 g of L-arginine hydrochloride in Example 7 was changed to 0.1 g of L-lysine. Table 2 presents the results.

### <Comparative Example 3>

Titanium oxide surface-treated with disodium N-stearoyl glutamate was obtained in the same manner as in Example 7 except that 0.1 g of L-arginine hydrochloride in Example 7 was changed to 0.1 g of L-aspartic acid. Table 2 presents the results.

### <Comparative Example 4>

Titanium oxide surface-treated with disodium N-stearoyl glutamate was obtained in the same manner as in Example 7 except that 0.1 g of L-arginine hydrochloride in Example 7 was changed to 0.1 g of L-glutamic acid. Table 2 presents the results.

**[Table 2]**

| Example/Comparative Example | | | Example 7 | Example 8 | Example 9 | Comparative Example 3 | Comparative Example 4 |
|---|---|---|---|---|---|---|---|
| Powder | | Titanium Oxide | 20 | 20 | 20 | 20 | 20 |
| Surface Treating Agent Composition | (a) | 2Na Stearoyl Glutamic Acid | 0.4 | 0.4 | 0.4 | 0.4 | 0.4 |
| | (b) | Glycerin | 1.2 | 1.2 | 1.2 | 1.2 | 1.2 |
| | (c) | Water | 1.2 | 1.2 | 1.2 | 1.2 | 1.2 |
| | (d) | L-Arg (Isoelectric Point 10.76) | 0.1 | | | | |
| | (d) | L-His (Isoelectric Point 7.59) | | 0.1 | | | |
| | (d) | L-Lys (Isoelectric Point 9.75) | | | 0.1 | | |
| | - | L-Aspartic Acid (Isoelectric Point 2.77) | | | | 0.1 | |
| | - | L-Glutamic Acid (Isoelectric Point 3.22) | | | | | 0.1 |
| Acidic Liquid | | 50% Citric Acid Liquid | 0.56 | 0.56 | 0.56 | 0.56 | 0.56 |
| Evaluation | | Evaluation of Oil Dispersibility Transmittance of Surface Treating Agent Composition (Wavelength of 660 nm) | A | B | B | E | E |
| | | | 90.6 | 90.4 | 90.8 | 90.4 | 91.0 |

### Examination of various blending amounts of the surface treating agent composition

### <Examples 10 to 22 and Comparative Example 5>

The blending amounts of the various components of Example 7 were changed as presented in Tables 3-1 and 3-2, and titanium oxide surface-treated with disodium N-stearoyl glutamate was obtained in the same manner as in Example 7. Tables 3-1 and 3-2 present the results.

In Comparative Example 5, since the amount of the surface treatment composition was too large with respect to the powder, it became a paste and the oil dispersibility was not evaluated.

**[Table 3-1]**

| Example/Comparative Example | | | Example | | | | | | | | |
|---|---|---|---|---|---|---|---|---|---|---|---|
| | | | 7 | 10 | 11 | 12 | 13 | 14 | 15 | 16 | 17 |
| Powder | | Titanium Oxide | 20 | 20 | 20 | 20 | 20 | 20 | 20 | 20 | 20 |
| Surface Treating Agent Composition | (a) | 2Na Stearoyl Glutamic Acid | 0.4 | 0.4 | 0.4 | 0.4 | 0.4 | 0.2 | 0.6 | 0.1 | 1 |
| | (b) | Glycerin | 1.2 | 1.2 | 1.2 | 1.2 | 1.2 | 1.2 | 1.2 | 1.2 | 1.2 |
| | (c) | Water | 1.2 | 1.2 | 1.2 | 1.2 | 1.2 | 1.2 | 1.2 | 1.2 | 1.2 |
| | (d) | L-Arg (Isoelectric Point 10.76) | 0.1 | 0.05 | 0.2 | 0.01 | 0.5 | 0.1 | 0.1 | 0.1 | 0.1 |
| Acidic Liquid | | 50% Citric Acid Liquid | 0.56 | 0.56 | 0.56 | 0.56 | 0.56 | 0.56 | 0.56 | 0.56 | 0.56 |
| Evaluation | | Evaluation of Oil Dispersibility | A | B | B | C | D | B | A | D | D |
| | | Amount (Mass) of Surface Treating Agent Composition | 2.9 | 2.85 | 3 | 2.81 | 3.3 | 2.7 | 3.1 | 2.6 | 3.5 |
| | | Transmittance of Surface Treating Agent Composition (Wavelength of 660 nm) | 90.6 | 90.2 | 91.4 | 65.4 | 32.1 | 95.3 | 93.2 | 97.5 | 1.4 |
| | | Amount of Surface Treating Agent Composition based on 100 Parts of Powder | 14.5 | 14.25 | 15 | 14.05 | 16.5 | 13.5 | 15.5 | 13 | 17.5 |
| | | Amount of Acid based on 100 Parts of Powder | 2.8 | 2.8 | 2.8 | 2.8 | 2.8 | 2.8 | 2.8 | 2.8 | 2.8 |
| | | Amount of Component (a) in Surface Treating Agent Composition | 13.8 | 14.0 | 13.3 | 14.2 | 12.1 | 7.4 | 19.4 | 3.8 | 28.6 |
| | | Amount of Component (b) in Surface Treating Agent Composition | 41.4 | 42.1 | 40.0 | 42.7 | 36.4 | 44.4 | 38.7 | 46.2 | 34.3 |
| | | Amount of Component (c) in Surface Treating Agent Composition | 41.4 | 42.1 | 40.0 | 42.7 | 36.4 | 44.4 | 38.7 | 46.2 | 34.3 |
| | | Amount of Component (d) in Surface Treating Agent Composition | 3.4 | 1.8 | 6.7 | 0.4 | 15.2 | 3.7 | 3.2 | 3.8 | 2.9 |

**[Table 3-2]**

| Example/Comparative Example | | | Example | | | | | Comparative Example | Reference Example | |
|---|---|---|---|---|---|---|---|---|---|---|
| | | | 18 | 19 | 20 | 21 | 22 | 5 | 1 | 2 |
| Powder | | Titanium Oxide | 20 | 20 | 20 | 20 | 1000 | 20 | 20 | 20 |
| Surface Treating Agent Composition | (a) | 2Na Stearoyl Glutamic Acid | 0.4 | 0.4 | 0.4 | 0.4 | 20 | 0.4 | 0.4 | 0.4 |
| | (b) | Glycerin | 1.92 | 1.44 | 0.96 | 0.48 | 60 | 2 | 2.4 | |
| | (c) | Water | 0.48 | 0.96 | 1.44 | 1.92 | 60 | 2 | | 2.4 |
| | (d) | L-Arg (Isoelectric Point 10.76) | 0.1 | 0.1 | 0.1 | 0.1 | 5 | 0.1 | 0.1 | 0.1 |
| Acidic Liquid | | 50% Citric Acid Liquid | 0.56 | 0.56 | 0.56 | 0.56 | 28 | 0.56 | 0.56 | 0.56 |
| Evaluation | | Evaluation of Oil Dispersibility | B | A | A | B | A | - | D | D |
| | | Amount (Mass) of Surface Treating Agent Composition | 2.9 | 2.9 | 2.9 | 2.9 | 145 | 4.5 | 2.9 | 2.9 |
| | | Transmittance of Surface Treating Agent Composition (Wavelength of 660 nm) | 83.2 | 88.3 | 89.2 | 80.1 | 90.6 | 96.5 | 32.4 | 2.1 |
| | | Amount of Surface Treating Agent Composition based on 100 Parts of Powder | 14.5 | 14.5 | 14.5 | 14.5 | 14.5 | 22.5 | 14.5 | 14.5 |
| | | Amount of Acid based on 100 Parts of Powder | 2.8 | 2.8 | 2.8 | 2.8 | 2.8 | 2.8 | 2.8 | 2.8 |
| | | Amount of Component (a) in Surface Treating Agent Composition | 13.8 | 13.8 | 13.8 | 13.8 | 13.8 | 8.9 | 13.8 | 13.8 |
| | | Amount of Component (b) in Surface Treating Agent Composition | 66.2 | 49.7 | 33.1 | 16.6 | 41.4 | 44.4 | 82.8 | 0.0 |
| | | Amount of Component (c) in Surface Treating Agent Composition | 16.6 | 33.1 | 49.7 | 66.2 | 41.4 | 44.4 | 0.0 | 82.8 |
| | | Amount of Component (d) in Surface Treating Agent Composition | 3.4 | 3.4 | 3.4 | 3.4 | 3.4 | 2.2 | 3.4 | 3.4 |

### (d) Comparison of components

### <Examples 23 to 25>

Titanium oxide surface-treated with disodium N-stearoyl glutamate was obtained in the same manner as in Example 15 except that 0.1 g of L-arginine hydrochloride of Example 15 was changed to various components (d2). Table 4 presents the results.

**[Table 4]**

| | | Example/Comparative Example | Example 15 | Example 23 | Example 24 | Example 25 |
|---|---|---|---|---|---|---|
| Powder | | Titanium Oxide | 20 | 20 | 20 | 20 |
| Surface Treating Agent Composition | (a) | 2Na Stearoyl Glutamic Acid | 0.6 | 0.6 | 0.6 | 0.6 |
| | (b) | Glycerin | 1.2 | 1.2 | 1.2 | 1.2 |
| | (c) | Water | 1.2 | 1.2 | 1.2 | 1.2 |
| | (d) | L-Arg (Isoelectric Point 10.76) | 0.1 | | | |
| | (d) | Sodium Aluminate | | 0.1 | | |
| | (d) | Magnesium Sulfate | | | 0.012 | |
| | (d) | Calcium Hydroxide | | | | 0.18 |
| Acidic Liquid | | 50% Citric Acid Liquid | 0.56 | | 0.56 | 0.56 |
| | | 64% Sulfuric Acid | | 0.06 | | |
| Evaluation | | Evaluation of Oil Dispersibility | | | | |
| | | Amount (Mass) of Surface Treating Agent Composition | A | A | A | A |
| | | Transmittance of Surface Treating Agent Composition (Wavelength of 660 nm) | 3.1 | 3.1 | 3.012 | 3.18 |
| | | Amount of Surface Treating Agent | 86.4 | 80.2 | 89.5 | 81.3 |
| | | Composition based on 100 Parts of Powder | | | | |
| | | Amount of Acid based on 100 Parts of Powder | 15.5 | 16.5 | 15.06 | 15.9 |
| | | Amount of Component (a) in Surface Treating Agent Composition | 2.8 | 0.3 | 2.8 | 2.8 |
| | | Amount of Component (b) in Surface Treating Agent Composition | 19.4 | 19.4 | 19.9 | 18.9 |
| | | Amount of Component (c) in Surface Treating Agent Composition | 38.7 | 38.7 | 39.8 | 37.7 |
| | | Amount of Component (d) in Surface Treating Agent Composition | 38.7 | 38.7 | 39.8 | 37.7 |

### Examination of the type of the powder to be treated

### <Examples 26 to 32>

Powders surface-treated with disodium N-stearoyl glutamate was obtained in the same manner as in Example 7 except that 20 g of titanium oxide in Example 7 (ISHIHARA SANGYO KAISHA, LTD., CR-50) was changed to the powders presented in Table 5. Table 5 presents the results of the oil dispersity evaluation of the surface-treated powders.

The details of the powders used are as follows.
Talc: Asada Seifun Co., Ltd., JA-46R
Mica: Yamaguchi Mica Co., Ltd., Y-2300X
Sericite: Sanshin Mining Ind. Co., Ltd., FSE
fine particle titanium oxide: ISHIHARA SANGYO KAISHA, LTD., TTO-55
Red iron oxide: Titan Kogyo, Ltd., R-516HP
Yellow iron oxide: Titan Kogyo, Ltd., LL-100HP
Black iron oxide: Titan Kogyo, Ltd., BL-100HP

**[Table 5]**

| | | Example/Comparative Example | Example | | | | | | |
|---|---|---|---|---|---|---|---|---|---|
| | | | 26 | 27 | 28 | 29 | 30 | 31 | 32 |
| Powder | | Talc | 20 | | | | | | |
| | | Mica | | 20 | | | | | |
| | | Sericite | | | 20 | | | | |
| | | Fine Particle Titanium Oxide | | | | 20 | | | |
| | | Red Iron Oxide | | | | | 20 | | |
| | | Yellow Iron Oxide | | | | | | 20 | |
| | | Black Iron Oxide | | | | | | | 20 |
| Surface Treating Agent Composition | (a) | 2Na Stearoyl Glutamic Acid | 0.4 | 0.4 | 0.4 | 0.4 | 0.4 | 0.4 | 0.4 |
| | (b) | Glycerin | 1.2 | 1.2 | 1.2 | 1.2 | 1.2 | 1.2 | 1.2 |
| | (c) | Water | 1.2 | 1.2 | 1.2 | 1.2 | 1.2 | 1.2 | 1.2 |
| | (d) | L-Arg (Isoelectric Point 10.76) | 0.1 | 0.1 | 0.1 | 0.1 | 0.1 | 0.1 | 0.1 |
| Acidic Liquid | | 50% Citric Acid Liquid | 0.56 | 0.56 | 0.56 | 0.56 | 0.56 | 0.56 | 0.56 |
| Evaluation | | Evaluation of Oil Dispersibility | A | A | A | A | B | B | A |
| | | Amount (Mass) of Surface Treating Agent Composition | 2.9 | 2.9 | 2.9 | 2.9 | 2.9 | 2.9 | 2.9 |
| | | Transmittance of Surface Treating Agent Composition (Wavelength of 660 nm) | 90.6 | 90.6 | 90.6 | 90.6 | 90.6 | 90.6 | 90.6 |

### Examination of the acid type and amount

### <Example 33>

Titanium oxide surface-treated with disodium N-stearoyl glutamate was obtained in the same manner as in Example 7 except that 0.56 g of 50% citric acid in Example 7 was changed to 2 g. Table 6 presents the results of the oil dispersity evaluation of the surface-treated powder.

### <Example 34>

Titanium oxide surface-treated with disodium N-stearoyl glutamate was obtained in the same manner as in Example 7 except that 0.56 g of 50% citric acid in Example 7 was changed to 0.01 g of 64% sulfuric acid. Table 6 presents the results of the oil dispersity evaluation of the surface-treated powder.

### <Example 35>

Titanium oxide surface-treated with disodium N-stearoyl glutamate was obtained in the same manner as in Example 7 except that 0.56 g of 50% citric acid in Example 7 was changed to 3.2 g. Table 6 presents the results of the oil dispersity evaluation of the surface-treated powder.

### <Example 36>

Titanium oxide surface-treated with disodium N-stearoyl glutamate was obtained in the same manner as in Example 7 except that 0.56 g of 50% citric acid in Example 7 was changed to 0.001 g of 64% sulfuric acid. Table 6 presents the results of the oil dispersity evaluation of the surface-treated powder.

**[Table 6]**

| Example/Comparative Example | | | Example | | | | |
|---|---|---|---|---|---|---|---|
| | | | 7 | 33 | 34 | 35 | 36 |
| Powder | | Titanium Oxide | 20 | 20 | 20 | 20 | 20 |
| Surface Treating Agent Composition | (a) | 2Na Stearoyl Glutamic Acid | 0.4 | 0.4 | 0.4 | 0.4 | 0.4 |
| | (b) | Glycerin | 1.2 | 1.2 | 1.2 | 1.2 | 1.2 |
| | (c) | Water | 1.2 | 1.2 | 1.2 | 1.2 | 1.2 |
| | (d) | L-Arg (Isoelectric Point 10.76) | 0.1 | 0.1 | 0.1 | 0.1 | 0.1 |
| Acidic Liquid | | 50% Citric Acid Liquid | 0.56 | 2 | | 3.2 | |
| | | 64% Sulfuric Acid | | | 0.01 | | 0.001 |
| Evaluation | | Evaluation of Oil Dispersibility | A | B | B | C | C |
| | | pH | 6.5 | 5.1 | 7.3 | 4.4 | 8.5 |
| | | Amount (Mass) of Surface Treating Agent Composition | 2.9 | 2.9 | 2.9 | 2.9 | 2.9 |
| | | Transmittance of Surface Treating Agent Composition (Wavelength of 660 nm) | 90.6 | 90.6 | 90.6 | 90.6 | 90.6 |
| | | Amount of Surface Treating Agent Composition based on 100 Parts of Powder | 14.5 | 14.5 | 14.5 | 14.5 | 14.5 |
| | | Amount of Acid based on 100 Parts of Powder | 2.8 | 10 | 0.05 | 16 | 0.005 |

### <Examples 37 to 47>

The blending amounts of the various components of Example 7 were changed as presented in Tables 7-1 and 7-2, and titanium oxide surface-treated with disodium N-stearoyl glutamate was obtained in the same manner as in Example 7. Tables 7-1 and 7-2 present the results.

**[Table 7-1]**

| Example/Comparative Example | | | Example | | | | | | | |
|---|---|---|---|---|---|---|---|---|---|---|
| | | | 7 | 37 | 38 | 39 | 40 | 41 | 42 | 43 |
| Powder | | Titanium Oxide | 20 | 20 | 20 | 20 | 20 | 20 | 20 | 20 |
| Surface Treating Agent Composition | (a) 2Na Stearoyl Glutamic Acid | | 0.4 | | | | | | | |
| | (a) Na stearoyl glutamic acid | | | 0.4 | 0.4 | 0.4 | 0.4 | 0.4 | 0.4 | 0.4 |
| | (a) stearoyl glutamic acid (Free Form) | | | | | | | | | |
| | (a) Na oleoyl glutamate (Monounsaturated) | | | | | | | | | |
| | (a) Na palmitoylglutamate | | | | | | | | | |
| | (a) Na myristoyl glutamate | | | | | | | | | |
| | (e) Na Stearate | | | | | | | | | |
| | (e) Na Palmitate | | | | | | | | | |
| | (b) Glycerin | | 1.2 | 1.2 | 1.2 | 1.2 | 1.2 | 1.2 | 1.2 | 1.2 |
| | (c) Water | | 1.2 | 0.95 | 0.7 | 0.45 | 0.4 | 0.35 | 0.2 | 0.2 |
| | (f) 1N NaOH | | | 0.25 | 0.5 | 0.75 | 0.8 | 0.85 | 1 | |
| | (f) 2N NaOH | | | | | | | | | 1 |
| | (d) L-Arg (Isoelectric Point 10.76) | | 0.1 | 0.1 | 0.1 | 0.1 | 0.1 | 0.1 | 0.1 | 0.1 |
| Acidic Liquid | | 50% Citric Acid Liquid | 0.56 | 0.56 | 0.56 | 0.56 | 0.56 | 0.56 | 0.56 | 0.56 |
| Evaluation | | Evaluation of Oil Dispersibility | A | D | B | A | A | A | D | D |
| | | Amount (Mass) of Surface Treating Agent Composition | 2.9 | 2.9 | 2.9 | 2.9 | 2.9 | 2.9 | 2.9 | 2.9 |
| | | Transmittance of Surface Treating Agent Composition (Wavelength of 660 nm) | 90.6 | 67.6 | 92.3 | 90.4 | 90.4 | 89.3 | 25.3 | 3.7 |
| | | Amount of Surface Treating Agent Composition based on 100 Parts of Powder | 14.5 | 14.5 | 14.5 | 14.5 | 14.5 | 14.5 | 14.5 | 14.5 |
| | | Amount of Acid based on 100 Parts of Powder | 2.8 | 2.8 | 2.8 | 2.8 | 2.8 | 2.8 | 2.8 | 2.8 |

**[Table 7-2]**

| Example/Comparative Example | | | Example | | | | | | |
|---|---|---|---|---|---|---|---|---|---|
| | | | 44 | 45 | 46 | 47 | 48 | 49 | 50 |
| Powder | | Titanium Oxide | 20 | 20 | 20 | 20 | 1000 | 1000 | 1000 |
| | (a) 2Na Stearoyl Glutamic Acid | | | | | | 20 | 12.35 | 10.66 |
| Surface | (a) Na stearoyl glutamic acid | | | | | | | | |
| Treating Agent | (a) stearoyl glutamic acid (Free Form) | | 0.4 | | | | | | |
| Composition | (a) Na oleoyl glutamate (Monounsaturated) | | | 0.4 | | | | | |
| | (a) Na palmitoylglutamate | | | | 0.4 | | | 6.65 | 5.74 |
| | (a) Na myristoyl glutamate | | | | | 0.4 | | | |
| | (e) Na Stearate | | | | | | | 0.65 | 2.34 |
| | (e) Na Palmitate | | | | | | | 0.35 | 1.26 |
| | (b) Glycerin | | 1.2 | 1.2 | 1.2 | 1.2 | 60 | 60 | 60 |
| | (c) Water | | 0.4 | 0.4 | 0.4 | 0.4 | 60 | 60 | 60 |
| | (f) 1N NaOH | | | 0.8 | 0.8 | 0.8 | | | |
| | (f) 2N NaOH | | 0.8 | | | | | | |
| | (d) L-Arg (Isoelectric Point 10.76) | | 0.1 | 0.1 | 0.1 | 0.1 | 5 | 5 | 5 |
| Acidic Liquid | | 50% Citric Acid Liquid | 0.56 | 0.56 | 0.56 | 0.56 | 28 | 28 | 28 |
| Evaluation | | Evaluation of Oil Dispersibility | A | A | B | B | A | A | A |
| | | Amount (Mass) of Surface Treating Agent Composition | 2.9 | 2.9 | 2.9 | 2.9 | 145 | 145 | 145 |
| | | Transmittance of Surface Treating Agent Composition (Wavelength of 660 nm) | 92.3 | 91.0 | 91.4 | 81.5 | 91.2 | 90.4 | 91 |
| | | Amount of Surface Treating Agent Composition based on 100 Parts of Powder | 14.5 | 14.5 | 14.5 | 14.5 | 14.5 | 14.5 | 14.5 |
| | | Amount of Acid based on 100 Parts of Powder | 2.8 | 2.8 | 2.8 | 2.8 | 2.8 | 2.8 | 2.8 |

### <Example 48>

In 60 g of glycerin and 60 g of water, 20 g of disodium N-stearoyl glutamate and 5 g of L-arginine hydrochloride were dissolved to obtain a surface treating agent composition. To FM Mixer (NIPPON COKE & ENGINEERING. CO., LTD., FM5C/Model I), 1000 g of titanium oxide (ISHIHARA SANGYO KAISHA, LTD., CR-50) was put and stirred at a blade rotation speed of 1500 rpm, and the surface treating agent composition was added thereto and mixed for 60 seconds. Then, 28 g of a 50% (w/v) citric acid aqueous solution was added and mixed for another 60 seconds to obtain titanium oxide surface-treated with disodium N-stearoyl glutamate without washing, filtering, or drying.

Further, 1000 g of titanium oxide was changed to 1000 g of ultra-fine particle titanium oxide (ISHIHARA SANGYO KAISHA, LTD., TTO-55 (N)), 1000 g of fine particle zinc oxide (TAYCA Co., Ltd., MZ-300), 1000 g of red iron oxide (Titan Kogyo, Ltd., R-516HP), 1000 g of yellow iron oxide (Titan Kogyo, Ltd., LL-100HP), 1000 g of black iron oxide (Titan Kogyo, Ltd., BL-100HP), 1000 g of talc (Asada Seifun Co., Ltd., JA-46R), 1000 g of mica (Yamaguchi Mica Co., Ltd., Y-2300X), and 1000 g of sericite (Sanshin Mining Ind. Co., Ltd., FSE), and the same method was used to obtain surface-treated ultra-fine particle titanium oxide, fine particle zinc oxide, red iron oxide, yellow iron oxide, black iron oxide, talc, mica, and sericite. Tables 7 and 8 present the results.

### <Example 49>

Surface-treated titanium oxide, ultra-fine particle titanium oxide, fine particle zinc oxide, red iron oxide, yellow iron oxide, black iron oxide, talc, mica, and sericite were obtained in the same manner as in Example 48 except that 20 g of disodium N-stearoyl glutamate was changed to 12.35 g of disodium N-stearoyl glutamate, 6.65 g of sodium N-palmitoylglutamate, 0.65 g of sodium stearate, and 0.35 g of sodium palmitate. Tables 7 and 8 present the results.

### <Example 50>

Surface-treated titanium oxide, ultra-fine particle titanium oxide, fine particle zinc oxide, red iron oxide, yellow iron oxide, black iron oxide, talc, mica, and sericite were obtained in the same manner as in Example 48 except that 20 g of disodium N-stearoyl glutamate was changed to 10.66 g of disodium N-stearoyl glutamate, 5.74 g of sodium N-palmitoylglutamate, 2.34 g of sodium stearate, and 1.26 g of sodium palmitate. Tables 7 and 8 present the results.

### <Comparative Example 6>

Titanium oxide of Comparative Example 1 at 20 g was changed to 20 g of ultra-fine particle titanium oxide (ISHIHARA SANGYO KAISHA, LTD., TTO-55 (N)), 20 g of fine particle zinc oxide (TAYCA Co., Ltd., MZ-300), 20 g of red iron oxide (Titan Kogyo, Ltd., R-516HP), 20 g of yellow iron oxide (Titan Kogyo, Ltd., LL-100HP), 20 g of black iron oxide (Titan Kogyo, Ltd., BL-100HP), 20 g of talc (Asada Seifun Co., Ltd., JA-46R), 20 g of mica (Yamaguchi Mica Co., Ltd., Y-2300X), and 20 g of sericite (Sanshin Mining Ind. Co., Ltd., FSE), and the same method was used to obtain surface-treated ultra-fine particle titanium oxide, fine particle zinc oxide, red iron oxide, yellow iron oxide, black iron oxide, talc, mica, and sericite. Table 8 presents the results.

**[Table 8]**

| | Evaluation of Oil Dispersibility |
|---|---|
| Titanium Oxide of Example 48 | A |
| Ultra-Fine Particle Titanium Oxide of Example 48 | A |
| Fine Particle Zinc Oxide of Example 48 | A |
| Red Iron Oxide of Example 48 | B |
| Yellow Iron Oxide of Example 48 | B |
| Black Iron Oxide of Example 48 | A |
| Talc of Example 48 | B |
| Mica of Example 48 | B |
| Sericite of Example 48 | A |
| Titanium Oxide of Example 49 | A |
| Ultra-Fine Particle Titanium Oxide of Example 49 | A |
| Fine Particle Zinc Oxide of Example 49 | A |
| Red Iron Oxide of Example 49 | A |
| Yellow Iron Oxide of Example 49 | A |
| Black Iron Oxide of Example 49 | A |
| Talc of Example 49 | A |
| Mica of Example 49 | B |
| Sericite of Example 49 | A |
| Titanium Oxide of Example 50 | A |
| Ultra-Fine Particle Titanium Oxide of Example 50 | A |
| Fine Particle Zinc Oxide of Example 50 | A |
| Red Iron Oxide of Example 50 | A |
| Yellow Iron Oxide of Example 50 | A |
| Black Iron Oxide of Example 50 | A |
| Talc of Example 50 | A |
| Mica of Example 50 | A |
| Sericite of Example 50 | A |
| Titanium Oxide of Comparative Example 1 | D |
| Ultra-Fine Particle Titanium Oxide of Comparative Example 6 | D |
| Fine Particle Zinc Oxide of Comparative Example 6 | C |
| Red Iron Oxide of Comparative Example 6 | C |
| Yellow Iron Oxide of Comparative Example 6 | C |
| Black Iron Oxide of Comparative Example 6 | C |
| Talc of Comparative Example 6 | C |
| Mica of Comparative Example 6 | C |
| Sericite of Comparative Example 6 | D |

### <Examples 101 to 103 and Comparative Examples 101 and 102>

Liquid foundations were prepared using the components presented in Table 9 as follows. The component B was dispersed at 6000 rpm for 5 minutes with Homo Mixer (Primix, MARK II Model 2.5). The component C was added thereto, and the mixture was dispersed at 4000 rpm for 5 minutes. While stirring at 3000 rpm, the completely dissolved component A was gradually added and emulsified to obtain a liquid foundation.

**[Table 9]**

| | Raw Material Name | Example 101 | Example 102 | Example 103 | Comparative Example 101 | Comparative Example 102 |
|---|---|---|---|---|---|---|
| A | Water | 45.4 | 45.4 | 45.4 | 45.4 | 45.4 |
| | 1,2-Pentanediol | 2 | 2 | 2 | 2 | 2 |
| | Sodium Chloride | 0.5 | 0.5 | 0.5 | 0.5 | 0.5 |
| | 2-Phenoxyethanol | 0.5 | 0.5 | 0.5 | 0.5 | 0.5 |
| B | Dimethicone | 16.5 | 16.5 | 16.5 | 16.5 | 16.5 |
| | Ethylhexyl Methoxycinnamate | 5 | 5 | 5 | 5 | 5 |
| | Isopropyl Lauroyl Sarcosinate | 5 | 5 | 5 | 5 | 5 |
| | PEG-20 Hydrogenated Castor Oil Triisostearate (HLB6) | 2 | 2 | 2 | 2 | 2 |
| | Trimethylsiloxysilicate | 3.5 | 3.5 | 3.5 | 3.5 | 3.5 |
| | Lauryl PEG-9 Polydimethylsiloxyethyl Dimethicone | 1 | 1 | 1 | 1 | 1 |
| | PEG-9 Polydimethylsiloxyethyl Dimethicone | 1 | 1 | 1 | 1 | 1 |
| | Vinyl Dimethicone/Methicone Silsesquioxane Crosspolymer | 1 | 1 | 1 | 1 | 1 |
| | N^{ε}-Lauroyl-L-lysine | 2 | 2 | 2 | 2 | 2 |
| | Titanium Oxide of Example 48 | 8 | - | - | - | - |
| | Red Iron Oxide of Example 48 | 0.2 | - | - | - | - |
| | Yellow Iron Oxide of Example 48 | 1.3 | - | - | - | - |
| | Black Iron Oxide of Example 48 | 0.1 | - | - | - | - |
| | Titanium Oxide of Example 49 | - | 8 | - | - | - |
| | Red Iron Oxide of Example 49 | - | 0.2 | - | - | - |
| | Yellow Iron Oxide of Example 49 | - | 1.3 | - | - | - |
| | Black Iron Oxide of Example 49 | - | 0.1 | - | - | - |
| | Titanium Oxide of Example 50 | - | - | 8 | - | - |
| | Red Iron Oxide of Example 50 | - | - | 0.2 | - | - |
| | Yellow Iron Oxide of Example 50 | - | - | 1.3 | - | - |
| | Black Iron Oxide of Example 50 | - | - | 0.1 | - | - |
| | Titanium Oxide (ISHIHARA SANGYO KAISHA, LTD., CR-50) | - | - | - | 8 | - |
| | Red Iron Oxide (Titan Kogyo, Ltd., R-516HP) | - | - | - | 0.2 | - |
| | Yellow Iron Oxide (Titan Kogyo, Ltd., LL-100HP) | - | - | - | 1.3 | - |
| | Black Iron Oxide (Titan Kogyo, Ltd., BL-100HP) | - | - | - | 0.1 | - |
| | Titanium Oxide of Comparative Example 1 | - | - | - | - | 8 |
| | Red Iron Oxide of Comparative Example 6 | - | - | - | - | 0.2 |
| | Yellow Iron Oxide of Comparative Example 6 | - | - | - | - | 1.3 |
| | Black Iron Oxide of Comparative Example 6 | - | - | - | - | 0.1 |
| C | Disteardimonium Hectorite | 5 | 5 | 5 | 5 | 5 |
| | Total (% by Mass) | 100 | 100 | 100 | 100 | 100 |

### <Test Example 1>

Evaluation of the concealability of powder dispersion:
According to the method using test plates, specified in 4.4 of JIS K 5600-4-1: 1999, the average value of three measurements using a fund-type cryptometer (manufactured by Coating Tester Co., Ltd.) was used as the concealability. The results in Table 10 present that the concealability of Examples and Comparative Examples was evaluated. It was confirmed that the concealability of Examples 101 to 103 was high, and the surface-treated powders of Examples 101 to 103 had high dispersibility in oil, preparing a liquid foundation with coverage.

**[Table 10]**

| | Concealability (m²/L) |
|---|---|
| Example 101 | 20.0 |
| Example 102 | 23.1 |
| Example 103 | 21.4 |
| Comparative Example 101 | 16.7 |
| Comparative Example 102 | 13.6 |

### <Test Example 2>

Sensory evaluation:
The liquid foundations of Examples 101 to 103 and Comparative Example 102 were sensory-evaluated by five expert panelists in terms of (1) smoothness (no roughness) during application, (2) elongation during application, (3) moistness after application, (3) dehydration freeness after application, and (5) adhesion to the skin. In the evaluation, each powder was applied to the inner side part of the forearm of the panelists, and the liquid foundation of Comparative Example 101 was used as a comparison target to evaluate each based on the following evaluation criteria. For each evaluation item, the average value M of the evaluation scores by the five people was calculated, which is presented in Table 13, with the case where 0.5 < M ≤ 2.0 being designated as "A," the case where 0 < M ≤ 0.5 as "B," the case where -0.5 < M ≤ 0 as "C," and the case where - 2.0≤ M ≤ -0.5 as "D."

### <Evaluation Criteria>

### (1) Smoothness (no roughness) during application

2 points: comfortably smooth compared to the comparison target
1 point: somewhat comfortably smooth compared to the comparison target
0 points: approximately the same level of smoothness compared to the comparison target
-1 points: slightly rough compared to the comparison target
-2 points: rough compared to the comparison target

### (2) Elongation during application

2 points: comfortable elongation compared to the comparison target
1 point: somewhat comfortable elongation compared to the comparison target
0 points: approximately the same level of elongation compared to the comparison target
-1 points: slightly sticky feeling compared to the comparison target
-2 points: sticky feeling compared to the comparison target

### (3) Moistness after application

2 points: comfortable moistness compared to the comparison target
1 point: somewhat comfortable moistness compared to the comparison target
0 points: approximately the same level of moistness compared to the comparison target
-1 points: slightly dry compared to the comparison target
-2 points: dry compared to the comparison target

### (4) Dehydration freeness after application

2 points: comfortable silkiness lasts compared to the comparison target
1 point: somewhat comfortable silkiness lasts compared to the comparison target
0 points: approximately the same level of silkiness lasts compared to the comparison target
-1 points: slightly dehydrated compared to the comparison target
-2 points: dehydrated compared to the comparison target

### (5) Adhesion to the skin

2 points: very high adhesion compared to the comparison target
1 point: somewhat high adhesion compared to the comparison target
0 points: approximately the same level of adhesion compared to the comparison target
-1 points: sits slightly bad on the skin compared to the comparison target
-2 points: sits bad on the skin compared to the comparison target

**[Table 11]**

| Evaluation Item | Example 101 | Example 102 | Example 103 | Comparative Example 102 |
|---|---|---|---|---|
| Smoothness (No Roughness) during Application | A | A | A | D |
| Slipperiness during Application | B | A | A | C |
| Moistness after Application | A | A | A | B |
| Dehydration Freeness after Application | B | A | B | B |
| Adhesion to Skin | A | B | B | C |

### <Examples 104 to 106 and Comparative Examples 103 and 104>

Pressed foundations were prepared using the components presented in Table 12 as follows. B was heated and dissolved. A was weighed and ground with a lab mixer. B was added to A and mixed with a lab mixer for another 5 minutes. It was passed through a sieve having a mesh size of 150 µm and compression-molded with a mold.

**[Table 12]**

| | Raw Material Name | Example 104 | Example 105 | Example 106 | Comparative Example 103 | Comparative Example 104 |
|---|---|---|---|---|---|---|
| A | Talc of Example 48 | 18.40 | - | - | - | - |
| | Sericite of Example 48 | 32.00 | - | - | - | - |
| | Mica of Example 48 | 20.00 | - | - | - | - |
| | Titanium Oxide of Example 48 | 8.00 | - | - | - | - |
| | Talc of Example 49 | - | 18.40 | - | - | - |
| | Sericite of Example 49 | - | 32.00 | - | - | - |
| | Mica of Example 49 | - | 20.00 | - | - | - |
| | Titanium Oxide of Example 49 | - | 8.00 | - | - | - |
| | Talc of Example 50 | - | - | 18.40 | - | - |
| | Sericite of Example 50 | - | - | 32.00 | - | - |
| | Mica of Example 50 | - | - | 20.00 | - | - |
| | Titanium Oxide of Example 50 | - | - | 8.00 | - | - |
| | Talc (JA-46R) | - | - | - | 18.40 | - |
| | Sericite (FSE) | - | - | - | 32.00 | - |
| | Mica (Y-2300X) | - | - | - | 20.00 | - |
| | Titanium Oxide (CR-50) | - | - | - | 8.00 | - |
| | Talc of Comparative Example 6 | - | - | - | - | 18.40 |
| | Sericite of Comparative Example 6 | - | - | - | - | 32.00 |
| | Mica of Comparative Example 6 | - | - | - | - | 20.00 |
| | Titanium Oxide of Comparative Example 1 | - | - | - | - | 8.00 |
| | Titanium Oxide, Al Stearate | 3.00 | 3.00 | 3.00 | 3.00 | 3.00 |
| | Zinc Oxide, Methicone | 2.00 | 2.00 | 2.00 | 2.00 | 2.00 |
| | Red Iron Oxide of Example 48 | 0.40 | - | - | - | - |
| | Yellow Iron Oxide of Example 48 | 1.00 | - | - | - | - |
| | Black Iron Oxide of Example 48 | 0.15 | - | - | - | - |
| | Red Iron Oxide of Example 49 | - | 0.40 | - | - | - |
| | Yellow Iron Oxide of Example 49 | - | 1.00 | - | - | - |
| | Black Iron Oxide of Example 49 | - | 0.15 | - | - | - |
| | Red Iron Oxide of Example 50 | - | - | 0.40 | - | - |
| | Yellow Iron Oxide of Example 50 | - | - | 1.00 | - | - |
| | Black Iron Oxide of Example 50 | - | - | 0.15 | - | - |
| | Red Iron Oxide (R-516HP) | - | - | - | 0.40 | - |
| | Yellow Iron Oxide (LL-100HP) | - | - | - | 1.00 | - |
| | Black Iron Oxide (BL-100HP) | - | - | - | 0.15 | - |
| | Red Iron Oxide of Comparative Example 6 | - | - | - | - | 0.40 |
| | Yellow Iron Oxide of Comparative Example 6 | - | - | - | - | 1.00 |
| | Black Iron Oxide of Comparative Example 6 | - | - | - | - | 0.15 |
| | N^{ε}-Lauroyl-L-lysine | 2.00 | 2.00 | 2.00 | 2.00 | 2.00 |
| | Methylparaben | 0.05 | 0.05 | 0.05 | 0.05 | 0.05 |
| | Nylon-12 | 2.00 | 2.00 | 2.00 | 2.00 | 2.00 |
| B | Dimethicone | 4.98 | 4.98 | 4.98 | 4.98 | 4.98 |
| | Polyglyceryl-2 Tetraisostearate | 3.00 | 3.00 | 3.00 | 3.00 | 3.00 |
| | Dipentaerythrityl Hexahydroxystearate/Hexastearate/Hexarosinate | 1.00 | 1.00 | 1.00 | 1.00 | 1.00 |
| | Mineral Oil | 2.00 | 2.00 | 2.00 | 2.00 | 2.00 |
| | Methylparaben | 0.01 | 0.01 | 0.01 | 0.01 | 0.01 |
| | Tocopherol | 0.01 | 0.01 | 0.01 | 0.01 | 0.01 |
| | Total (% by Mass) | 100.0 | 100.0 | 100.0 | 100.0 | 100.0 |

### <Test Example 3>

Sensory evaluation:
The pressed foundations of Examples 104 to 106 and Comparative Example 104 were sensory-evaluated by five expert panelists in terms of (1) smoothness (no roughness) during application, (2) elongation during application, (3) moistness after application, (3) dehydration freeness after application, and (5) adhesion to the skin. In the evaluation, each powder was applied to the inner side part of the forearm of the panelists, and the pressed foundations of Comparative Example 103 was used as a comparison target to evaluate each based on the following evaluation criteria. For each evaluation item, the average value M of the evaluation scores by the five people was calculated, which is presented in Table 13, with the case where 0.5 < M ≤ 2.0 being designated as "A," the case where 0 < M ≤ 0.5 as "B," the case where -0.5 < M ≤ 0 as "C," and the case where - 2.0≤ M ≤ -0.5 as "D."

### <Evaluation Criteria>

### (1) Smoothness (no roughness) during application

2 points: comfortably smooth compared to the comparison target
1 point: somewhat comfortably smooth compared to the comparison target
0 points: approximately the same level of smoothness compared to the comparison target
-1 points: slightly rough compared to the comparison target
-2 points: rough compared to the comparison target

### (2) Elongation during application

2 points: comfortable elongation compared to the comparison target
1 point: somewhat comfortable elongation compared to the comparison target
0 points: approximately the same level of elongation compared to the comparison target
-1 points: slightly sticky feeling compared to the comparison target
-2 points: sticky feeling compared to the comparison target

### (3) Moistness after application

2 points: comfortable moistness compared to the comparison target
1 point: somewhat comfortable moistness compared to the comparison target
0 points: approximately the same level of moistness compared to the comparison target
-1 points: slightly dry compared to the comparison target
-2 points: dry compared to the comparison target

### (4) Dehydration freeness after application

2 points: comfortable silkiness lasts compared to the comparison target
1 point: somewhat comfortable silkiness lasts compared to the comparison target
0 points: approximately the same level of silkiness lasts compared to the comparison target
-1 points: slightly dehydrated compared to the comparison target
-2 points: dehydrated compared to the comparison target

### (5) Adhesion to the skin

2 points: very high adhesion compared to the comparison target
1 point: somewhat high adhesion compared to the comparison target
0 points: approximately the same level of adhesion compared to the comparison target
-1 points: sits slightly bad on the skin compared to the comparison target
-2 points: sits bad on the skin compared to the comparison target

**[Table 13]**

| Evaluation Item | Example 104 | Example 105 | Example 106 | Comparative Example 104 |
|---|---|---|---|---|
| Smoothness (No Roughness) during Application | A | A | A | C |
| Slipperiness during Application | B | A | B | B |
| Moistness after Application | A | A | A | B |
| Dehydration Freeness after Application | A | A | B | C |
| Adhesion to Skin | A | A | A | C |

### <Example 107 and Comparative Example 105>

Lip cosmetics were prepared using the components presented in Table 14 as follows. A was heated and dissolved at 105°C. B was added, and the mixture was heated and dissolved at 90°C. C was further added, and the mixture was heated and mixed at 90°C. D, which had been previously dispersed by three rollers, was added thereto. After adding E and heating and mixing at 90°C, defoaming was performed. The mixture was filled in a sink at a filling temperature of 90°C, rapidly cooled at -5°C, returned to room temperature, and loaded in a product container.

Compared with Comparative Example 105, the lip cosmetic of Example 107 had better color development, less color unevenness, and better color uniformity. Furthermore, there was no sweating or blooming, and the storage stability was good.

**[Table 14]**

| | Raw Material Name | Example 107 | Comparative Example 105 |
|---|---|---|---|
| A | Polyethylene | 2.8 | 2.8 |
| | Triethylhexanoin | 15 | 15 |
| B | Candelilla Wax | 1 | 1 |
| | Paraffin | 7 | 7 |
| | Microcrystalline Wax | 6 | 6 |
| | Hydrogenated Polyisobutene | 12 | 12 |
| | Dipentaerythrityl Hexahydroxystearate/Hexastearate/Hexarosinate | 5 | 5 |
| | Isotridecyl Isononanoate | 10 | 10 |
| | Trimethylolpropane Triisostearate | 5 | 5 |
| | Tocopherol | 0.1 | 0.1 |
| | Methylparaben | 0.5 | 0.5 |
| | Isopropyl Lauroyl Sarcosinate | 5 | 5 |
| C | Titanium Oxide, Mica, Silica | 4 | 4 |
| | Mica, Titanium Oxide | 2 | 2 |
| D | Red 201 | 0.27 | 0.27 |
| | Red 202 | 0.54 | 0.54 |
| | Red Iron Oxide of Example 49 | 0.60 | - |
| | Yellow Iron Oxide of Example 49 | 0.10 | - |
| | Titanium Oxide of Example 49 | 1 | - |
| | Red Iron Oxide of Comparative Example 6 | - | 0.60 |
| | Yellow Iron Oxide of Comparative Example 6 | - | 0.10 |
| | Titanium Oxide of Comparative Example 1 | - | 1 |
| | Polyglyceryl-2 Triisostearate | 3 | 3 |
| E | Polyglyceryl-2 Triisostearate | 12.59 | 12.59 |
| | Total (% by Mass) | 100 | 100 |

### <Example 108>

An eye shadow was prepared as follows using the components presented in Table 15. The component A was mixed well with a blender, and the component B, which had been uniformly dissolved in advance, was added and mixed. It was sieved with a mesh size of 150 µm and compression-molded by a press machine.

The eye shadow of Example 108 did not impair the luster of the raw material of the pearl pigment itself even after press molding, and maintained the luster during use. Furthermore, it was excellent in color development.

**[Table 15]**

| | Raw Material Name | Example 108 |
|---|---|---|
| A | Talc of Example 49 | 45 |
| | Mica of Example 49 | 14 |
| | Sericite of Example 49 | 5 |
| | Black Iron Oxide of Example 49 | 12 |
| | Yellow Iron Oxide of Example 49 | 1 |
| | Red Iron Oxide of Example 49 | 2 |
| | Titanium Oxide, Talc | 10 |
| | Methylparaben | 0.5 |
| B | Liquid Paraffin | 6 |
| | Methylpolysiloxane | 2 |
| | Sorbitan Sesquioleate | 2 |
| | Tocopherol | 0.5 |
| | Total (% by Mass) | 100 |

### <Example 109>

A pencil composition was prepared using the components presented in Table 16 as follows. The component A was mixed with FM Mixer (NIPPON COKE & ENGINEERING. CO., LTD., FM5C/Model I) at 1000 rpm for 5 minutes. The mixed component A was added to the component B previously dissolved at 95°C, kneaded well, and kneaded with a vacuum extrusion molding machine. It was molded into a core with an extrusion molding machine and sandwiched between shaft plates to form a pencil shape.

The pencil of the Example had a hard core to break and had a high gloss. Furthermore, it was excellent in color development.

**[Table 16]**

| | Raw Material Name | Example 109 |
|---|---|---|
| A | Black Iron Oxide of Example 49 | 20 |
| | Titanium Oxide of Example 49 | 3 |
| | Talc of Example 49 | 7 |
| | Talc, Titanium Oxide | 5 |
| | Kaolin | 15 |
| B | Japan Wax | 20 |
| | Stearic Acid | 10 |
| | Beeswax | 5 |
| | Hardened Castor Oil | 5 |
| | Vaseline | 4 |
| | Lanolin | 3 |
| | Liquid Paraffin | 2.9 |
| | Tocopherol | 0.1 |
| | Total (% by Mass) | 100 |

### <Example 110>

An antiperspirant composition was prepared using the components presented in Table 17 as follows. A was heated and dissolved at 95°C and then allowed to cool to 75°C. B was heated and dissolved at 70°C, A was added, and the mixture was thoroughly mixed. After adding C and mixing, the mixture was filled in a product container and cooled to room temperature.

The antiperspirant of Example 110 had less stickiness and white residue, and was good in elongation enough to be applied thinly and evenly. In addition, the storage stability was also good.

**[Table 17]**

| | Raw Material Name | Example 110 |
|---|---|---|
| A | Dibutyl Ethylhexanoyl Glutamide | 3 |
| | Dibutyl Lauroyl Glutamide | 5 |
| | Propylene Glycol | 6 |
| | Hexildecanol | 18 |
| B | Cyclopentasiloxane | Balance |
| | PPG-14 Butyl Ether | 7 |
| | Al Chlorohydrate | 16 |
| | Talc | 1.2 |
| | N^{ε}-Lauroyl-L-lysine | 0.5 |
| | Silica | 0.1 |
| C | Tocopherol | 0.1 |
| | Total (% by Mass) | 56.9 |

### <Example 51>

Silica surface-treated with disodium N-stearoyl glutamate was obtained in the same manner as in Example 7 except that 20 g of titanium oxide in Example 7 was changed to 20 g of silica (P-1500, JGC Catalysts and Chemicals Ltd.). Also, similarly, cornstarch surface-treated with disodium N-stearoyl glutamate was obtained in the same manner as in Example 7 except that 20 g of titanium oxide in Example 7 was changed to 20 g of cornstarch (Japanese Pharmacopoeia Corn Starch ST-C, NIPPON STARCH CHEMICAL CO., LTD.). Further, carbon black surface-treated with disodium N-stearoyl glutamate was obtained in the same manner as in Example 7 except that 20 g of titanium oxide in Example 7 was changed to 20 g of carbon black (MIDNIGHT BLACK, GEOTECH International B.V).

### <Examples 111 and 112 and Comparative Example 106>

Leave-on cosmetics were prepared using the components presented in Table 18 as follows. The component B was thoroughly mixed. The components C and D were added in order and dissolved at 85°C. Next, component E was added and dissolved at 85°C. The component G and the component H that had been mixed in advance were added to prepare an aqueous phase. Separately, the component A was mixed at 85°C, the aqueous phase was added while stirring with Homo Mixer (Primix, MARK II Model 2.5), and the mixture was stirred at 85°C for 3 minutes (5000 rpm). Then, using a mechanical stirrer, the mixture was cooled to 45°C with stirring at 50 rpm. Further, using a mechanical stirrer, the component F was added and mixed for 1 minute while stirring at 100 rpm. It was allowed to stand at room temperature to obtain a leave-on cosmetic. Compared with Comparative Example 106, the leave-on cosmetic of Example 111 was excellent in smoothness at the time of application and clearness of the skin, and the gloss was further improved by application. Further, compared with Comparative Example 106, the leave-on cosmetic of Example 112 was excellent in moistness after application and clearness of the skin, and the gloss was further improved by application.

**[Table 18]**

| | Raw Material Name | Example 111 | Example 112 | Comparative Example 106 |
|---|---|---|---|---|
| A | Squalane | 5 | 5 | 5 |
| | Jojoba Oil | 5 | 5 | 5 |
| | Macadamia Nut Oil | 5 | 5 | 5 |
| | Capric/Caprylic Triglyceride | 5 | 5 | 5 |
| | Phytosteryl/Octyldodecyl Lauroyl Glutamate | 1 | 1 | 1 |
| | Isostearyl Hydroxystearate | 2 | 2 | 2 |
| | Shea Butter | 2 | 2 | 2 |
| | Stearyl Alcohol | 3.8 | 3.8 | 3.8 |
| | Carnauba Wax | 0.1 | 0.1 | 0.1 |
| | Glyceryl Stearate | 2.9 | 2.9 | 2.9 |
| | N^{ε}-Lauroyl-L-lysine | 0.3 | 0.3 | 0.3 |
| | Silica of Example 51 | 2 | - | - |
| | Cornstarch of Example 51 | - | 2 | - |
| | Nylon-12 | - | - | 2 |
| B | Xanthan Gum | 0.2 | 0.2 | 0.2 |
| | Water | 49.5 | 49.5 | 49.5 |
| C | Fragrance | 0.3 | 0.3 | 0.3 |
| D | 10% Sodium Hydroxide Aqueous Solution | 1 | 1 | 1 |
| E | Sucrose Palmitate | 0.4 | 0.4 | 0.4 |
| F | 8.26% Citric Acid Aqueous Solution | 4.29 | 4.29 | 4.29 |
| | Na Citrate | 0.21 | 0.21 | 0.21 |
| G | 3% Sodium Stearoyl Glutamate Aqueous Solution | 5 | 5 | 5 |
| H | 1% PCA Ethyl Cocoyl Arginate Aqueous Solution | 5 | 5 | 5 |
| | Total (% by Mass) | 100 | 100 | 100 |

### <Example 113>

A facial cleansing powder was prepared using the components presented in Table 19 as follows. All components were stirred and mixed with a mixer for 1 minute. The obtained facial cleansing powder had good water compatibility and foaming, and after facial cleansing, the skin did not become stiff and the moist feel continued.

**[Table 19]**

| Raw Material Name | Example 113 |
|---|---|
| Sodium Lauroyl Glutamate | 15 |
| Sodium Myristoyl Glutamate | 15 |
| Mannitol | 10 |
| Talc of Example 49 | 20 |
| Cornstarch of Example 51 | 23.5 |
| Glucose | 15 |
| N^{ε}-Lauroyl-L-lysine | 1 |
| Agar | 0.5 |
| Total (% by Mass) | 100 |

### <Example 114>

A mascara was prepared using the components presented in Table 20 as follows. The component A was dispersed, and when hydroxyethyl cellulose was completely hydrated, it was heated to 80°C. The component C was weighed and heated to 80°C to melt all the components. The component B was gradually added to the component C, and the mixture was dispersed at 6000 rpm for 5 minutes with Homo Mixer (Primix, MARK II Model 2.5). It was allowed to cool to room temperature to obtain the mascara. The obtained mascara had good color development, no lumpy pigment, and good adhesion to the lashes.

**[Table 20]**

| | Raw Material Name | Example 114 |
|---|---|---|
| A | 0.1% Xanthan Gum Aqueous Solution | 64.8 |
| | Hydroxyethyl Cellulose | 0.2 |
| | Hydrogenated Polyisobutene | 3 |
| | Polyvinylpyrrolidone | 2 |
| | 1,3-Butylene Glycol | 2 |
| | Ethanol | 2 |
| B | Black Iron Oxide of Example 49 | 5 |
| | Carbon Black of Example 51 | 5 |
| | N^{ε}-Lauroyl-L-lysine | 5 |
| C | Beeswax | 2 |
| | Carnauba Wax | 3 |
| | Paraffin | 4 |
| | Phenoxy ethanol | 1 |
| | Methylparaben | 0.5 |
| | Propyl Paraben | 0.5 |
| | Total (% by Mass) | 100 |

### <Example 115 and Comparative Example 107>

W/O type emulsion type suntan lotion cosmetic products were prepared using the components presented in Table 21 as follows. The component A and component B were heated to 70°C and dissolved. The dissolved powder component of the component A was sufficiently dispersed, and the dissolved component B was added while treating with Homo Mixer (Primix, MARK II Model 2.5) at 4000 rpm. The emulsion was stirred to room temperature while being cooled with water to obtain a W/O type emulsion type suntan lotion cosmetic product. Compared to Comparative Example 107, the W/O type emulsion type suntan lotion cosmetic product of Example 115 was hard to run off by sweat and water and thus water-resistant, less sticky with a fresh feel, and good in elongation on the skin, with sufficient ultraviolet protection effect.

**[Table 21]**

| | Raw Material Name | Example 115 | Comparative Example 107 |
|---|---|---|---|
| | | | |
| A | Octyl Paramethoxycinnamate | 5.0 | 5.0 |
| | Oxybenzone | 3.0 | 3.0 |
| | 4-tert Butyl-4'-Methoxybenzoylmethane | 1.0 | 1.0 |
| | Ultra-Fine Particle Titanium Oxide of Example 48 | 5.0 | - |
| | Fine Particle Zinc Oxide of Example 48 | 5.0 | - |
| | Ultra-Fine Particle Titanium Oxide of Comparative Example 6 | - | 5.0 |
| | Fine Particle Zinc Oxide of Comparative Example 6 | - | 5.0 |
| | Squalane | 20.0 | 20.0 |
| | Isopropyl Lauroyl Sarcosinate | 3.0 | 3.0 |
| | Silicone Oil | 20.0 | 20.0 |
| | Silicone Resin | 2.0 | 2.0 |
| | Glycerin Diisostearate | 2.0 | 2.0 |
| | Bentonite | 0.5 | 0.5 |
| | Methylparaben | 0.5 | 0.5 |
| | Propyl Paraben | 0.5 | 0.5 |
| B | Water | 27.5 | 27.5 |
| | 1,3-Butylene Glycol | 5.0 | 5.0 |
| | Total (% by Mass) | 100 | 100 |

### <Example 116 and Comparative Example 108>

W/O type cream type suntan lotion cosmetic products were prepared using the components presented in Table 22 as follows. The component A and component B were heated to 70°C and dissolved. The dissolved powder component of the component A was sufficiently dispersed, and the dissolved component B was added while treating with Homo Mixer (Primix, MARK II Model 2.5) at 4500 rpm. The emulsion was stirred to room temperature while being cooled with water to obtain a W/O type cream type suntan lotion cosmetic product. Compared to Comparative Example 108, the W/O type cream type suntan lotion cosmetic product of Example 116 was excellent in storage stability, hard to run off by sweat and water and thus water-resistant, fresh in feel, and good in elongation on the skin, with sufficient ultraviolet protection effect.

**[Table 22]**

| | Raw Material Name | Example 116 | Comparative Example 108 |
|---|---|---|---|
| A | Ultra-Fine Particle Titanium Oxide of Example 49 | 10.0 | - |
| | Fine Particle Zinc Oxide of Example 50 | 10.0 | - |
| | Ultra-Fine Particle Titanium Oxide of Comparative Example 6 | - | 10.0 |
| | Fine Particle Zinc Oxide of Comparative Example 6 | - | 10.0 |
| | Squalane | 17.0 | 17.0 |
| | Isopropyl Lauroyl Sarcosinate | 3.0 | 3.0 |
| | Silicone Oil | 12.0 | 12.0 |
| | Glycerin Diisostearate | 3.0 | 3.0 |
| | Organically Modified Monomorilonite | 1.5 | 1.5 |
| | Methylparaben | 0.5 | 0.5 |
| | Propyl Paraben | 0.5 | 0.5 |
| B | Water | 37.5 | 37.5 |
| | 1,3-Butylene Glycol | 5.0 | 5.0 |
| | Total (% by Mass) | 100 | 100 |

## Claims

1. A surface treatment method for a powder comprising the steps of:
(i) mixing 0.001 to 20 parts by mass of a surface treating agent composition based on 100 parts by mass of the powder; and
(ii) adding an acidic liquid, followed by mixing, wherein
the surface treating agent composition contains the following components (a) to (d)
(a) at least one selected from N-acyl amino acids having a carbon chain length of C8 to C22 and salts thereof,
(b) at least one selected from monohydric and polyhydric alcohols,
(c) water, and
(d) at least one selected from the following (d1) and (d2)
(d1) amino acids having an isoelectric point of 7.5 to 11 and
(d2) compounds that produce ions selected from aluminum ions, magnesium ions, calcium ions, zinc ions, zirconia ions, and titanium ions in the surface treating agent composition.

2. The method according to claim 1, wherein in the step (i), the surface treating agent composition is mixed by dropping or mixed by spraying.

3. The method according to claim 1 or 2, wherein in the step (i), the surface treating agent composition is mixed in an amount of 0.1 to 20 parts by mass based on 100 parts by mass of the powder.

4. The method according to any one of claims 1 to 3, wherein in the steps (i) and (ii), the mixing is performed using a mixer selected from the group consisting of high-speed stirring type mixers, container rotary type mixers, container rotary type mixers with a stirrer, mechanical stirring type mixers, airflow stirring type mixers, and compression/shear/impact type mixers.

5. The method according to any one of claims 1 to 4, wherein the acidic liquid is a citric acid aqueous solution or sulfuric acid.

6. The method according to any one of claims 1 to 5, wherein in the step (ii), the acidic liquid is mixed in an amount of 0.01 to 15 parts by mass based on 100 parts by mass of the powder.

7. The method according to any one of claims 1 to 6, wherein the powder is at least one selected from the group consisting of resin powders, silicon-containing powders, metal oxides, carbon-containing powders, fluorine-containing powders, metal salts, boron-containing powders, composite powder crystals, and non-crystalline powders.

8. The method according to any one of claims 1 to 7, which is free of a drying step using power and an apparatus.

9. A surface treating agent composition comprising the following components (a) to (d):
(a) at least one selected from N-acyl amino acids having a carbon chain length of C8 to C22 and salts thereof;
(b) at least one selected from monohydric and polyhydric alcohols;
(c) water; and
(d) at least one selected from the following (d1) and (d2)
(d1) amino acids having an isoelectric point of 7.5 to 11 and
(d2) compounds that produce ions selected from aluminum ions, magnesium ions, calcium ions, zinc ions, zirconia ions, and titanium ions in the surface treating agent composition.

10. The surface treating agent composition according to claim 9, wherein a transmittance at a wavelength of 660 nm is 80% or more.

11. The surface treating agent composition according to claim 9 or 10, wherein a concentration of (a) in the surface treating agent composition is 4 to 25% by mass.

12. The surface treating agent composition according to any one of claims 9 to 11, wherein a concentration of (b) in the surface treating agent composition is 3 to 80% by mass.

13. The surface treating agent composition according to any one of claims 9 to 12, wherein a concentration of (c) in the surface treating agent composition is 3 to 80% by mass.

14. The surface treating agent composition according to any one of claims 9 to 13, wherein a concentration of (d) in the surface treating agent composition is 0.5 to 15% by mass.

15. The surface treating agent composition according to any one of claims 9 to 14, wherein a solid content concentration in the surface treating agent composition is 10 to 23% by mass.

16. The surface treating agent composition according to any one of claims 9 to 15, wherein (a) in the surface treating agent composition includes at least one selected from myristoyl glutamic acid, sodium myristoyl glutamate, palmitoylglutamic acid, sodium palmitoylglutamate, stearoyl glutamic acid, sodium stearoyl glutamate, disodium stearoyl glutamate, and sodium oleoyl glutamate.

17. The surface treating agent composition according to any one of claims 9 to 16, wherein (b) in the surface treating agent composition includes at least one selected from ethanol, 2-propanol, glycerin, pentanediol, dipropylene glycol, 1,3-butylene glycol, and pentylene glycol.

18. The surface treating agent composition according to any one of claims 9 to 17, wherein (d) in the surface treating agent composition includes at least one amino acid selected from histidine, lysine, and arginine.

19. The surface treating agent composition according to any one of claims 9 to 18, further comprising: at least one selected from sodium hydroxide, potassium hydroxide, sodium carbonate, potassium carbonate, and sodium hydrogen carbonate.

20. The surface treating agent composition according to any one of claims 9 to 19, further comprising: at least one selected from fatty acids having a carbon chain length of C8 to C22 and salts thereof.

21. A powder treated by the method according to any one of claims 1 to 8.

22. A cosmetic powder comprising, on a surface thereof:
(a) at least one selected from N-acyl amino acids having a carbon chain length of C8 to C22 and salts thereof; and
(d1) at least one selected from amino acids having an isoelectric point of 7.5 to 11.

23. The cosmetic powder according to claim 22, further comprising, on the surface thereof: (b) at least one selected from monohydric and polyhydric alcohols.

24. A cosmetic composition comprising: the powder according to any one of claims 21 to 23.
